# EUROPEAN PATENT SPECIFICATION

(11) **EP 1 613 180 B1**
(45) Date of publication and mention of the grant of the patent: **23.01.2008**
(21) Application number: 04726152.4
(22) Date of filing: 07.04.2004
(51) Int. Cl.: A23L 1/30

(54) **SYNBIOTIC COMBINATION**
SYMBIOTISCHE ZUSAMMENSETZUNG
COMBINAISON SYMBIOTIQUE

(30) Priority: 08.04.2003 GB 0308104
(43) Date of publication of application: 11.01.2006
(73) Proprietor: Nestec S.A., 1800 Vevey (CH); The University of Reading, Reading RG6 6AP (GB)
(72) Inventor: BEER, Michael, CH-3098 Köniz (CH); GIBSON, Glenn R., Reading, Berkshire RG2 9DW (GB); SMEJKA, Christopher, Redruth, Cornwall TR16 5JN (GB)
(74) Representative: Lock, Graham James
(86) International application number: PCT/EP2004/003736
(87) International publication number: WO 2004/089115

(56) References cited:
- EP-A- 1 010 372
- EP-A- 1 195 095
- WO-A-03/033681
- US-B1- 6 203 797
- DATABASE WPI Section Ch, Week 200301 Derwent Publications Ltd., London, GB; Class B04, AN 2003-013662 XP002290436 & RU 2 190 015 C1 (PARTNER STOCK CO) 27 September 2002 (2002-09-27)
- OKKERS D J ET AL: "CHARACTERIZATION OF PENTOCIN TV35B, A BACTERIOCIN-LIKE PEPTIDE ISOLATED FROM LACTOBACILLUS PENTOSUS WITH A FUNGISTATIC EFFECT ON CANDIDA ALBICANS" November 1999 (1999-11), JOURNAL OF APPLIED MICROBIOLOGY, OXFORD, GB, PAGE(S) 726-734 , XP009006853 ISSN: 1364-5072 abstract; figure 4; table 1

## Description

The invention relates to nutritional and pharmaceutical compositions comprising a probiotic and a prebiotic, e.g. non-digestible oligosaccharide, and in particular to synbiotic compositions; to medical applications of such compositions in promoting gut health, in particular in stimulating the immune system, in combating Candidiasis, in aiding digestion in general, and in alleviating the symptoms of Irritable Bowel Syndrome (IBS); and to a method for the production of such compositions.

Irritable bowel syndrome (IBS) is characterized by abdominal pain and discomfort, bloating, and altered bowel function, constipation and/or diarrhea. There are three groups of IBS: Constipation predominant IBS (C-IBS), Alternating IBS (A-IBS) and Diarrhea predominant IBS (D-IBS). The prevalence of IBS differs by country, however recent studies suggest that the disorder affects approximately about 25% of the Western population. IBS is recognized as a chronic condition, which may have profound effect on patients' quality of life. There is an urgent need for effective nutritional approaches capable of treating or preventing or ameliorating the effects of IBS.

Probiotics are microorganisms such as lactobacilli and bifidobacteria which beneficially affect the host by improving its intestinal microbial balance, and aiding digestion. Research has shown that probiotic microorganisms can influence the composition of the gut microflora by out-competing out pathogenic bacteria and yeasts. Probiotics are also alleged to stimulate the immune system, for example in patients with milk allergy, by reducing gut permeability and by enhancing local intestinal lymphoid cells involved in immune responses. Use of probiotics have been advocated for prophylaxis and treatment of diarrhea and for enhancing the recovery of the commensal microflora after antibiotic therapy. Anticarcinogenic activity has also been demonstrated in clinical trials with probiotics.

A number of "live" dairy products on the market claim to contain particularly high counts of certain probiotic Lactobacilli or Bifidobacteria species for promotion of digestion and a healthy immune system. However, the full range of desirable probiotic effects is exhibited by only a selected few strains of bacteria. It must also be borne in mind that the survivability of a strain in the gastrointestinal (GI) tract is crucial for the biological efficacy of that strain in situ. In order for the strain to implant and establish itself in the intestine it should ideally adhere to the mucosal surface of the GI tract and be able to survive the rigours of transit, such as exposure to stomach and bile acids.

Because forexample Bifidobacteria are susceptible to oxygen and heat, their application in foods has been limited. Therefore, there has been an increased interest in probiotics, which show effectiveness and endure normal food processing.

Prebiotics are non-digestible food ingredients, e. g.oligosaccharides, which have a beneficial effect on health. Fora food ingredient to be classified as a prebiotic it must fulfill the following criteria: i) neither be hydrolyzed, nor absorbed in thegastrointestinal tract, ii) be selectively fermented by one or a limited number of potentially beneficial bacteria commensal to the colon, such as lactobacilli and bifidobacteria, which are stimulated to grow and/or become metabolically activated, iii) be able to alter the colonic microflora towards a healthier composition, by increasing, for example, numbers ofsaccharolytic species while reducing putrefactive microorganisms such as bacteroides.

A desirable attribute for prebiotics is the ability to persist towards the distal region of the colon. This region of the gut is the site of origin of several chronic disease states including colon cancer and ulcerative colitis. It is thought that themicroflora in this region of the gut may play an important role in the onset or maintenance of such disorders. Dietary carbohydrate is the main fermentable substrate in the proximal colon and as this is degraded during bacterial fermentation, protein takes over as the dominant fermentable substrate towards more distal regions. The products of bacterial protein metabolism include toxic and potentially carcinogenic compounds such as amines, ammonia and phenolic compounds.

In WO 03/033681, a Lactobacillus strain showing the characteristic features of the strain deposited under accession number NCIMB 41114 has been disclosed, moreover fiber/dietary fiber prebiotic components have been generally mentioned.

RU 2 190 015 discloses Lactobacillus brevis Ba-13 use for probiotic and food stuff preparations. The strain suppresses growth of Candida Species and is resistant to tetracyclines.

Present inventors have now surprisingly found that a mixture of a particular probiotic with prebiotics has the ability to beneficially affect the host by improving the survival and implantation of live microbial in the GI tract, enhancing thesurvivability of the probiotic and also increasing the beneficial microorganisms in the gut.

Hence, the present invention pertains toa composition comprising the novel probiotic strain of Lactobacillus, deposited 28 August 2001 according to the Budapest Treaty at the NCIMB, 23 St. Machar Drive, Aberdeen AB243RY, Scotland, UK, under the accession number NCIMB 41114, and one or more prebiotic(s), e.g. one or more non-digestible oligosaccharide(s), hereinafter referred to as the compositions of the invention.

The compositions of the invention may be useful in promoting gut health, in particular in stimulating the immune system, in combating Candidiasis, in aiding digestion in general, and in alleviating the symptoms of Irritable Bowel Syndrome (IBS).

16S rRNA sequencing was carried out on the product and the sequencing of a 1500 bp test yielded a sequence as described in the Examples hereinbelow. This is one of the characterizing features of the strain deposited under the accession number NCIMB 41114 and disclosed in International Patent Application no. EP02/11428, which is hereby incorporated by reference.

In addition to exhibiting characteristics typical of Lactobacilli in general, Lactobacillus strain NCIMB 41114 has a further surprising property: it is capable of suppressing the growth of Candida species to a degree never previously achieved through use of a probiotic. Furthermore, tetracycline and related antibiotics have no effect on growth of this strain. This unique combination of properties can be exploited to combat undesirable growth of Candida in any region of the body. In particular, since Candida is considered to be a causative factor in irritable Bowel Syndrome (IBS), it is envisaged that this novel strain of Lactobacillus could be employed in treating or preventing that disorder.

As used herein, the terms "Lactobacillus strain NCIMB 41114" or "Lactobacillus strain of the invention" refer to the Lactobacillus strain deposited under the accession number NCIMB 41114 and equivalent Lactobacillus strains. Lactobacillus strain of the invention can be live, attenuated or killed.

As used herein, the term "oligosaccharide" refers to a saccharide consisting of at least two, up to 20 glycosidically linked monosaccharide units, i.e. having a degree of polymerization (DP) of 2 to 20, preferably of 2 to 15 monosaccharide units, more preferably of 2 to 10 monosaccharide units, and even more preferably of 2 to 7 monosaccharide units. According to the invention synthetic oligosaccharides or oligosaccharides isolated from natural sources may be used.

For the purpose of the present invention, the term "non-digestible oligosaccharides" encompasses oligosaccharides, e.g. synthetic or isolated from natural sources, which are not readily converted to caloric substrates by the human digestive enzymes.

The composition of the invention may comprise one or several non-digestible oligosaccharides, which may provide for health promoting action in different parts of the gastrointestinal tract enhancing beneficial bacteria.

Examples of suitable non-digestible oligosaccharides according to the present invention include fructo-oligosaccharides (FOS), galacto-oligosaccharides (GOS), lactulose, xylo-oligosaccharides (XOS), isomalto-oligosaccharides (IMO), ABG, soybean-oligosaccharide (soy-oligosaccharides, SOS), gentio-oligosaccharide, gluco-oligosaccahride, fructans, lactosucrose, short chain FOS and mixtures thereof, preferably FOS, GOS, XOS, short chain FOS, e.g. with less than 5 monosaccharide untis. According to the invention, in particular GOS and/or FOS may be used.

Fructo-oligosaccharides, also known as oligofructose, (FOS) are indigestible oligosaccharides that are members of the inulin subclass of fructans. FOS occurs in nature in many kinds of plants, including onions, garlic, shallots, wheat, rye, bananas, asparagus, tomatoes, artichokes, dahlia and chicory root. FOS can be produced enzymatically, through chemical techniques or by extraction from natural substances. Short chain FOS are composed of one to three fructose molecules linked to one molecule of sucrose: their polymerization degree (DP) is not higher than 6, e.g. not higher than 5, and they can be synthesized from sucrose through the use of transfructosylating enzymes. Treatment of sucrose with these transfructosylating enzymes results in a mixture of FOS containing 2, 3 or 4 fructose units, such as 1-kestose, nystose and fructosyl-nystose.

As used herein the term "FOS" encompass FOS and short chain FOS, e.g. having a polymerization degree (DP) not higher than 6, preferably not higher than 5. According to the invention, FOS may comprise between 2 and 20 saccharide units, preferably between 2 to 15 saccharide units, more preferably between 2 to 7 saccharide units and even more preferably between 2 to 6 saccharide units. In one embodiment of the invention, FOS may contain about 95% by weight disaccharides to heptasaccharides, based on the total weight of FOS. Preferably, FOS may comprise less then 10%, e.g. less than 5% by weight hepta- to octa-saccharides based on the total weight of FOS.

Oligofructose is commercially available, for example as RAFTILOSE P95, e.g. from ORAFTI, Tienen, Belgium, in various grades such as, for example, RAFTILOSE P95 which contains about 95 % by weight oligofructose, composed of chains with a degree of polymerisation ranging from 2 to about 7, typically with a DP of 3.5 to 4.5, and containing about 5 % by weight in total of glucose, fructose and sucrose.

Galacto-oligosaccharides (GOS) may comprise di, tri, tetra, penta and hexasaccharides, mainly consisting of galactose as a sugar component, and are formed by the action of beta-galactosidase on lactose. According to the invention, GOS may comprise between 2 and 15 saccharide units, preferably between 2 to 10 saccharide units, more preferably between 2 to 7 saccharide units and even more preferably between 2 to 6 saccharide units. In one embodiment of the invention, GOS may contain about 0 to about 45% of weight disaccharides, preferably about 10 to about 40% of weight disaccharides, more preferably about 20 to about 35% of weight disaccharides, and even more preferably about 33% of weight disaccharides, based of the total weight of GOS. According to the invention, GOS may contain about 0 to about 50% of weight trisaccharides, preferably about 10 to about 45% of weight trisaccharides, more preferably about 20 to about 40% of weight trisaccharides, and even more preferably about 39% of weight trisaccharides, based of the total weight of GOS. According to the invention, GOS may contain about 0 to about 50% of weight tetrasaccharides, preferably about 5 to about 45% of weight tetrasaccharides, more preferably about 10 to about 40% of weight tetrasaccharides, and even more preferably about 18% of weight tetrasaccharides, based of the total weight of GOS. According to the invention, GOS may contain about 0 to about 30% of weight pentasaccharides, preferably about 1 to about 25% of weight pentasaccharides, more preferably about 2 to about 10% of weight pentasaccharides, and even more preferably about 7% of weight pentasaccharides, based of the total weight on GOS.

Gluco-oligosaccharide may be produced from the sucrose by using the glucosyl-transferase from *Leuconostoc mesenteroides,* which transfers glucose molecules from a sucrose donor to a maltose acceptor. The gluco-oligosaccharide thus obtained is a mixture of different sized oligosaccharides. Gluco-oligosaccharide may also be extracted from oat beta-glucans.

The relative proportion of the active ingredients of the compositions of the invention will, of course, vary considerably depending on the particular type of composition concerned, e.g. whether it is a liquid or solid form, or whether it is provided in nutritional form. All indicated proportions and relative weight ranges described below are accordingly to be understood as being indicative of preferred or individually inventive teaching only and not limiting the invention in its broadest aspect.

Accordingly, suitable amounts of non-digestible oligosaccharides comprised in ready-for-consumption compositions according to the invention, e.g. ready-to-drink compositions or instant drink, are in the range of up to about 60% by weight, for example up to about 50% by weight, further example up to about 45% by weight, or from about 2.5 to about 35% by weight, for example from about 5 to about 30% by weight, further example from about 10 to about 25% by weight, based on the total weight of the ready-for-consumption composition.

The compositions of the invention in powder form may comprise non-digestible oligosaccharides in an amount of up to 95% by weight, e.g. about 5 to about 90% by weight, more preferably in an amount of about 10 to about 85% by weight, more preferably in an amount of about 15 to about 80% by weight and even more preferably in an amount of about 20 to about 75% by weight, based on the total weight of the powder composition.

An effective amount of non-digestible oligosaccharides may be a daily dosage of about 0.5 to about 40 g of non-digestible oligosaccharides, preferably about 1 to about 35 g, more preferably about 2 to about 30 g, even more preferably about 3 to about 30 g, for example about 10 g or about 20g.

According to the invention, the non-digestible oligosaccharides may be administered to an adult in an amount between about 0.1 and about 0.5 g per kg body weight per day, for example between about 0.2 and about 0.4 g per kg body weight per day, and to an infant in an amount between abut 0.2 and 1.0 g per kg body weight per day.

The Lactobacillus strain of the invention can be cultured on a large scale, e.g. using skimmed milk as media, e.g. maintained at a pH of above 5.5, and used therapeutically in the compositions of the invention in lyophilized, freeze-dried, spray-dried or hydrated form. It may also be used in the compositions of the invention in conjunction with a pharmaceutically acceptable carrier or nutritional matrix.

The amount of probiotic, e.g. lyophilized bacteria, incorporated into the ready-for-consumption compositions of the invention may vary from about 1 to about 30% by weight, preferably from about 2 to about 20% by weight, even more preferably from about 4 to about 10% by weight, based on the total weight of the composition. The concentration of probiotic incorporated into the ready-for-consumption compositions of the invention may lie in the range of 10⁶ to 10¹² cfu/g (colony forming units/g), preferably in the range of 10⁷ to 0.5x10¹², more preferably in the range of 10⁹ to 10¹¹, particularly preferred in the range of 0.5x10¹⁰ to 1.5x10¹⁰ or 2x10¹⁰ cfu/g, e.g. about 10¹⁰ cfu/g. For example, 1 to 5 g, e.g. 2g spray dried Lactobacillus strain NCIMB 41114, e.g. comprising about 10⁶ to about 10¹² cfu/g, e.g. about 10¹⁰ cfu/g may be administered per day. It is preferred that the bacterial strain is administered in live form, but prior to consumption the bacteria may be attenuated or killed.

In one embodiment of the invention, the compositions of the invention are in powder form comprising the probiotic in an amount of from about 0.05 to 90% by weight, preferably from about 0.5 to about 80% by weight, more preferably from about 20 to about 60% by weight of the composition, based on the total weight of the composition.

In another embodiment of the invention, the weight ratio of probiotic to prebiotic, e.g. non-digestible oligosaccharides, in the compositions of the invention, in powder or in ready for consumption form, may be from about 1 : 5 to about 5:1, e.g. from about 1 : 4 to about 4: 1 or from about 1: 3 to about 3:1.

According to the invention, the daily dosage of probiotic may be from of 5x10⁸ to 1x10¹² cfu (colony forming units), preferably 1x10⁹ to 5x10¹¹ cfu, a particularly preferred daily dosage may be about 10¹⁰, 2x10¹⁰ or 10¹¹ cfu.

In one aspect of the invention, the total amount of probiotic and prebiotic, e.g. non-digestible oligosaccharide in the compositions of the invention, in powder or in ready for consumption form, may be from about 1 to about 100% by weight, more preferably from about 5 to about 90% by weight, even more preferably from 10 to 80% by weight, based on the total weight of the composition.

In one embodiment of the invention, compositions of the invention may consist exclusively or essentially of the probiotic and prebiotic(s), e.g. non-digestible oligosaccharide(s). Alternatively, the compositions of the invention may comprise, in addition to the probiotic and non-digestible oligosaccharide(s), other nutritional components like fat, carbohydrate, minerals and vitamins, for example calcium, magnesium, iron, zinc, phosphorus, vitamin D and/or vitamin K. Further, the nutritional composition of the invention may contain one or more of the following ingredients in addition to the probiotic and non-digestible oligosaccharide(s): sucrose, dextrose, fructose, dried honey, dried fruit sugars, oligosaccharides, vegetable fibers, full cream or skimmed milk powder, milk proteins, vegetable starch, flour or protein, cocoa powder, nuts, chocolate, coffee, vanilla, lecithin, salt, flavours, colours.

The compositions of the invention may comprise one or more fibers. Fibers in particular include soluble and insoluble non-digestible polysaccharides. As used herein, the term "soluble fiber" refers to fibers which are able to undergo fermentation in the colon to produce short chain fatty acids (SCFA). Suitable examples of soluble fibres are: pectin, guar gum, e.g. hydrolyzed guar gum, e.g. partially hydrolyzed guar gum, and gum arabic.
The hydrolyzed soluble fiber may be derived from numerous known soluble fibers, including locust bean gum, xanthan gum, guar gum, and pectin. Preferably, hydrolyzed guar gum, e.g. partially hydrolyzed guar gum or hydrolyzed pectin may be used. The term "hydrolyzed soluble fibers" as used herein refers to soluble fibers hydrolyzed in a conventional manner, e.g. chemically or enzymatically to soluble fibers that have a reduced molecular weight. Such hydrolyzed products are, for example, tube compatible when administered at the desired daily amount.

A particularly preferred hydrolyzed soluble fiber may be hydrolyzed guar gum, e.g. as known and commercially available under the tradename Benefiber^{®}, e.g. as described in U.S. Patent No. 5,260,279, which is hereby incorporated by reference. Prior to hydrolysis, the molecular weight of guar gum is approximately 200,000; after hydrolysis it is 20,000-30,000.

For use in accordance with this invention, the molecular weight range of the hydrolyzed guar gum may vary, preferably may be between 24 and 30 kDa.

According to the invention, the non-digestible oligosaccharide, e.g. GOS and/or FOS, and the soluble fiber, e.g. hydrolyzed soluble fiber, e.g. partially hydrolyzed guar gum, may be used in a ratio of non-digestible oligosaccharide : hydrolyzed soluble fiber of about 3 : 1 to about 1 : 3, e.g. in a ratio of about 3 : 1.

In one embodiment of the invention, compositions of the invention may also comprise one or more fatty acids, for example polyunsaturated fatty acids. As used herein, the term cis-polyunsaturated fatty acid refers to a family of carboxylic acids comprising n-3 fatty acids such as alpha-linolenic acid (18:3), stearidonic acid, eicosapentaenoic acid (EPA) (20:5), docosapentaenoic acid (22:5) and docosahexaenoic acid (DHA) (22:6), and n-6 fatty acids such as linoleic acid (18:2), conjugated linoleic acid, gamma-linolenic acid (18:3), arachidonic acid (20:4), either in free form or in form of an oil or fat. Such cis-polyunsaturated fatty acids are commonly known and readily commercially available. They are present for example in vegetable oils or fish oils, e.g. concentrated fish oils, e.g. comprising of about 70% of eicosapentaenoic acid.
Preferably a combination of eicosapentaenoic acid and docosahexaenoic acid may be used.

Further components of the compositions according to the invention may include any bioactive compounds or extracts which are known to have health benefits, especially compounds which have a beneficial influence on the gastro-intestinal tract, such as glutamine/glutamate or precursors thereof. The compositions of the invention may also contain one or more additional substances that inhibit bacterial adhesion to epithelial wall of the gastrointestinal tract, including mannans, galacturonic acid oligomers, preferably of natural origin. The composition of the invention may be combined with drugs useful for the treatment of ulcerative colitis, such as sulphasalazine, 5-ASA agents, corticosteroids, such as adrenal steroids, prednisone, hydrocortisone or budesonide; or drugs used against pain, diarrhea, infection or IBS, such as a serotonin-4 receptor agonist, e.g. tegaserod, e.g. as known and commercially available under the trademarks Zelnorm™/Zelmac™. For example, the composition of the invention may be provided in the form of a kit for separate, sequential or simultaneous administration in conjunction with such medicines as described hereinabove. These medicines may conveniently be formulated together with the composition of the invention in standard pharmaceutical dosage forms, e.g. in combination with at least one pharmaceutically acceptable carrier.

In yet a further aspect the compositions as described herein may be combined with bioactive compounds, extracts or drugs which are known to have gastrointestinal side effects, e.g. diarrhea, for example with anti-cancer drugs such as epothilone.

In another aspect of the invention the compositions of the invention may be administered separately with the medicines hereinabove decribed in form of a co-therapy. For example, the compositions according to the invention may be readily incorporated into nutritional formulations, typically nutraceuticals, dietary supplements, functional food and beverage products, e.g. in combination with at least one nutritionally acceptable carrier.

In another aspect of the invention, there is provided a medicament, pharmaceutical or nutritional formulation, for example dietary supplement, comprising the composition of the invention. The medicament, pharmaceutical or nutritional composition of the invention may optionally comprise pharmaceutically or nutritionally acceptable carriers.

Further, according to the invention there is provided a combined pharmaceutical preparation, e.g. a commercial package, comprising as active agents Lactobacillus strain NCIMB 41114 or an equivalent Lactobacillus strain and a non-digestible oligosaccharide, e.g. an oligosaccharide chosen from at least one of galacto-oligosaccharide (GOS), and fructo-oligosaccharide (FOS), xylo-oligosaccharide (XOS), soybean-oligosaccharide (SOS), isomalto-oligosaccharide (IMO), gentio-oligosaccharide, lactulose, gluco-oligosaccharide, lactosucrose, fructan or inulin, together with instructions for simultaneous, separate or sequential use thereof in maintaining and/or restoring a beneficial gut microflora, for the prevention or treatment of any form of Functional Bowel Disorder (FBD), and in particular Irritable Bowel Syndrome (IBS), for eliminating sulfate reducing bacteria, for the treatment or prevention of Inflammatory Bowel Disease (IBD), in particular Ulcerative Colitis, Crohn's disease, and/or colon cancer, and/or for repressing or prolonging the remission periods on Ulcerative patients.

Pharmaceutical compositions and dietary supplements may be provided in the form of soft gel, sachets, powders, syrups, liquid suspensions, emulsions and solutions in convenient dosage forms. In soft capsules the active ingredients are preferably dissolved or suspended in suitable liquids, such as fatty oils, paraffin oil or liquid polyethylene glycols. Optionally stabilisers may be added.

Oral pharmaceutical or dietary supplement forms may be made by conventional compounding procedures known in the pharmaceutical art, that is, by mixing the active substances together with edible pharmaceutically acceptable solid or liquid carriers and/or excipients, e.g. fillers such as cellulose, lactose, sucrose, mannitol, sorbitol, and calcium phosphates and binders, such as starch, gelatin, tragacanth, methylcellulose and/or polyvinylpyrrolidone (PVP). Optional additives include lubricants and flow conditioners, e.g. silicic acid, silicon dioxide, talc, stearic acid, magnesium/calcium stearates, polyethylene glycol (PEG) diluents, disintegrating agents, e.g. starch, carboxymethyl starch, cross-linked PVP, agar, alginic acid and alginates, colouring agents, flavouring agents, and melting agents. Dyes or pigments may be added to the tablets or dragée coatings, for example for identification purposes or to indicate different doses of active ingredient.

Optionally, the compositions according to the invention may be nutritionally complete, i.e. may include vitamins, minerals, trace elements as well as nitrogen, carbohydrate and fatty acid sources so that they may be used as the sole source of nutrition supplying essentially all the required daily amounts of vitamins, minerals, carbohydrates, fatty acids, proteins and the like. Accordingly, the compositions of the invention may be provided in the form of a nutritionally balanced complete meal, e.g. suited for oral feeding.

Alternatively, the compositions of the invention may be provided as part of a meal, i.e. a nutritional supplement, e.g. in the form of a health drink.

The compositions of the invention may optionally comprise of conventional food additives, such as any of emulsifiers, stabilizers, sweeteners, flavourings, colouring agents, preservatives, chelating agents, osmotic agents, buffers or agents for pH adjustment, acidulants, thickeners and texturisers.

Suitable product formats according to the present invention include solution, ready-for-consumption composition, e.g. ready-to-drink compositions, instant drink, liquid comestibles, like soft drinks, juice, sports drinks, milk drinks, milk-shakes, yogurt drinks or soup. In a further embodiment of the invention, the compositions of the present invention may be manufactured and sold in the form of a concentrate, a powder, or granules, e.g. effervescent granules, which are diluted with water or other liquid, such as milk or fruit juice, e.g. orange juice, to yield a ready-for-consumption composition, e.g. ready-to-drink compositions or instant drink.

The compositions of the invention may further be incorporated, e.g. dispersed, in foods of any sort, such as, dairy bars, breakfast cereals, müesli, candies, tablets, cookies, biscuits, crackers, such as a rice crackers.

The composition of the invention may be in any form suitable for human administration, and in particular for administration in any part of the gastrointestinal tract. Enteral administration of the compositions of the invention, preferably oral administration, and administration through a tube or catheter, are covered by the present invention.

The amount and dosage regimen of the compositions of the invention to be administered is determined in the light of various relevant factors including the purpose of administration, the age, sex and body weight of individual subject and the severity of the subject's symptoms. For example, the dose of probiotic per serving may be between about 10⁶ to 10¹², or 5x10⁸ to 1x10¹² cfu, e.g. about 10¹⁰, 2x10¹⁰, or 10¹¹ cfu. The dose of prebiotic per serving may be between about 5 to 15 e.g. 10 g. According to the invention, up to 3, e.g. about 1 or 2 servings may be given per day.

The compositions of the invention may be administered under the supervision of a medical specialist, e.g. when co-administered with the medicines described herein above, or may be self-administered.

The compositions of the invention may be administered daily, weekly or monthly, or may be administered annually or even for longer periods. Suitable daily dosage regimen may include single or multiple servings per day. Optimally, the composition of the invention is consumed at least once a day on a regular basis, prior to, i.e. pre or post-prandial administration, or during a meal. In one embodiment of the invention, the composition of the invention may be taken until for example an improvement in their symptoms, e.g. constipation or diarrhea is observed. Since these formulations are safe to consume, subjects with IBS can continue taking the composition according to the invention for as long as required, and preferably until a marked relief from symptoms is experienced.

Pharmaceutical formulations, food or beverages incorporating the compositions according to the invention can be safely-consumed by anyone, and are especially recommended for anyone suffering from a disturbed intestinal function, or perceived to be at risk from FBD (Functional Bowel Disorder) or IBS, such as the elderly, menopausal women or clinical patients. The compositions of the invention may be particularly suitable for subjects suffering from intestinal or digestive troubles, Crohn's disease, intestinal diverticula or carcinoma of colon.

In another embodiment, the present invention relates to a process for the production of the composition of the invention, wherein such a process comprises intimately admixing the components of the composition of the invention. Such processes are well known to one skilled in the art.

By out-competing the harmful bacteria for growth in the intestines, in particular the large intestine, and more particularly the colon, and restoring or promoting a healthy balance of gut bacteria, the compositions of the invention may reduce the toxic effects on the digestive process, stimulate the digestive system and improve bowel control. The compositions of the invention may be effective in reducing the frequency, recurrence, severity, and duration of attacks of diarrhea or constipation. When consumed regularly the compositions of the invention may reduce the risk of an individual developing chronic symptoms, namely clinical disorders of the bowel.

The compositions of the invention may be effective to treat or prevent any form of Functional Bowel Disorder (FBD), and in particular Irritable Bowel Syndrome (IBS), such as Constipation predominant IBS (C-IBS), Alternating IBS (A-IBS) and Diarrhea predominant IBS (D-IBS); functional constipation and functional diarrhea. FBD is a general term for a range of gastrointestinal disorders which are chronic or semi-chronic and which are associated with bowel pain, disturbed bowel function and social disruption. Particular combinations and prevalence of symptoms characterize the following seven FBD subgroups, which are defined in accordance with the classification system known as the "Rome criteria": 1) C1: Constipation-predominant Irritable Bowel Syndrome ; 2) C1: Diarrhea-predominant Irritable Bowel Syndrome ; 3) C3: Functional constipation ; 4) C4: Functional diarrhea; 5) C2: Functional abdominal bloating; 6) F3a: Pelvic Floor dyssynergia; 7) F3b: Internal Anal Sphincter Dysfunction.

IBS may be characterized by at least 3 continuous months or recurrent symptoms of:
1. abdominal pain or discomfort which is (a) relieved with defecation, (b) and/or associated with a change in frequency of stool, (c) and/or associated with a change in consistency of stool; and
2. two or more of the following, on at least a quarter of occasion or days;
(a) altered stool frequency, (b) altered stool form (lumpy/hard or loose/watery), (c) altered stool passage (straining, urgency, or feeling of incomplete evacuation), (d) passage of mucus, (e) bloating or feeling of abdominal distention.

Functional Constipation is defined by two or more of the following symptoms for at least 3 months:
1. straining at defecation at least a quarter of the time; 2. lumpy and/or hard stools at least a quarter of the time; 3. sensation of incomplete evacuation at least a quarter of the time; 4. two or fewer spontaneous bowel movements in a week. Abdominal pain is not required, loose stools are not present, and there are insufficient criteria for IBS. These criteria may not apply when the patient is taking laxatives.

Functional Diarrhea is characterized by two or more of the following symptoms for at least 3 months: 1. unformed (mushy or watery) stool more than three quarters of the time; 2. three or more bowel movements per day greater than half the time; and 3. increased stool bowel as compared to the community norm (>200g/day for North Americans and Europeans) but no more than 500g per day. There are insufficient criteria for IBS. Abdominal pain is not complained of, and hard or lumpy stools are not present. Urgency is a prominent symptom and fecal incontinence or soiling may occur.

Similarly, functional abdominal bloating is typified by symptoms of abdominal fullness, bloating or distention. Pelvic Floor Dyssynergia is characterised by straining and a feeling of incomplete evacuation. Internal Anal Sphincter Dysfunction is diagnosed by Pelvic Floor Dyssynergia symptoms together with manometric tests.

In one embodiment of the invention, the compositions according to the invention may be used in the manufacture of a medicament or nutritional formulation for the prevention or treatment of gastrointestinal functional or organic disorders including constipation, diarrhea or any form of Functional Bowel Disorder (FBD), and in particular irritable bowel syndrome (IBS), such as Constipation predominant IBS (C-IBS), Alternating IBS (A-IBS) and Diarrhea predominant IBS (D-IBS); functional constipation and functional diarrhea.

In another embodiment of the invention, the compositions of the invention may be used in the manufacture of a medicament or nutritional formulation for controlling the pH in the colon, for controlling colonic motion and transit time, as anti-carcinogenic, anti-mutagenic, or hypocholesterolemic agent.

In another embodiment of the invention, the compositions according to the invention may be used in the manufacture of a medicament or nutritional formulation for stimulating the immune system and for promoting resistance to bacterial or yeast infections, in particular Candidiasis or diseases induced by sulfate reducing bacteria.

In another embodiment of the invention, the compositions according to the invention may be used in the manufacture of a medicament or nutritional formulation for the prevention or treatment of diseases, conditions and symptoms related to IBD, in particular Ulcerative Colitis, Crohn's disease or colon cancer in mammal, including human.

According to the invention, the compositions of the invention may have a bifidogenic effect. They may be used to promote a healthy flora in the gastrointestinal tract, which in infants makes it more similar to the flora of breast-fed infants and/or may be used to prevent and/or treat any disturbance in the naturally occurring flora in the gastrointestinal tract.

According to the invention, there is provided a method of treatment or prevention of a functional bowel disorder, such as IBS, e.g. constipation-predominant Irritable Bowel Syndrome, Diarrhea-predominant Irritable Bowel Syndrome; Functional Constipation; or Functional diarrhea, comprising administering to a patient in need of such treatment a composition of the invention.

In a further aspect there is provided a method for the dietary management of gastrointestinal functional or organic disorders or diseases, e.g. conditions and symptoms related to functional bowel disorder, such as IBS, e.g. constipation-predominant Irritable Bowel Syndrome, Diarrhea-predominant Irritable Bowel Syndrome; Functional Constipation; Functional diarrhea; or conditions and symptoms related to IBD, e.g. Ulcerative Colitis; Crohn's disease or colon cancer, comprising administering to a patient in need of such treatment a composition of the invention.

Pharmaceutical formulations, food or beverages incorporating the composition according to the invention can be safely-consumed by anyone, and are especially recommended for anyone perceived to be at risk or suffering from gastrointestinal functional or organic disorders or diseases, e.g. conditions and symptoms related to IBS, or IBD, in particular Ulcerative Colitis , Crohn's disease or colon cancer.

In one embodiment of the invention, the invention pertains to a method of treating and/or preventing diseases, conditions and symptoms related to IBS, in particular such forms of IBS as hereinabove described, in a mammal, including human, in need of such a treatment, comprising administering to said mammal an effective amount of a composition according to the invention. As used herein, the term "an effective amount" refers to an amount effective to achieve a desired therapeutic effect, such as treating and/or preventing diseases, conditions and symptoms related to IBS, in particular as hereinabove described.

The compositions of the invention are stable, preferably have a shelf life of at least six months at room temperature (18-22°C). The compositions of the invention do not need to be stored refrigerated. Dependent on the product format the compositions of the invention may be stored refrigerated, e.g. when in form of milk based products, e.g. yoghurts. According to the invention it is possible to effectively ameliorate symptoms and conditions associated with IBS with compositions which do not show any severe side effects. For example, the present methods are well-tolerated, simple to apply, and improve the quality of life of subjects with any form of Functional Bowel Disorder (FBD).

The utility of all the combinations of the present invention may be observed in standard clinical tests in, for example, known indications, for example using nutritional compositions as described in the Examples hereinbelow, for example using Lactobacillus strain NCIMB 41114 in the range of from about 5x10⁸ to 1x10¹² cfu e.g. about 10¹⁰ or 10¹¹ cfu, and one or more prebiotics, e.g. GOS, in a range of from about 5 to 15, e.g. 10 g, for a mammal, e.g. adult and in standard animal models. The relief in symptoms characterizing FBD provided by the compositions may be observed in standard animal tests and in clinical trials, e.g. as described hereinbelow.

The beneficial effects of the composition of the invention may be tested in the gut model as follows:
A three stage continuous culture system is set up in order to check the survivability of the probiotic strain in the gut and the effect of the different prebiotics on the gut microflora and on the growth of the probiotic strain.
The gut model is run using the faecal sample of an IBS patient as an inoculum with a retention time of 72h. After reaching the steady state treatment with the combination of the prebiotic and the probiotic is started. The prebiotic is added to the media in a concentration of e.g. about 4-5gr per day. The probiotic is added every day in a concentration of e.g. about 10⁸ cells/ml until the steady state (7 turnovers). Samples are taken every week for enumeration of the probiotic, for enumeration of different bacterial groups (fluorescent in situ hybridisation, F.I.S.H.), short chain fatty acid analysis and selective plating are used for screening the changes in the predominant bacterial species that will grow in the system. Finally the gas production is measured before and during the treatment with the synbiotic. At the same time a gut model with the same conditions is run using the faecal sample of a healthy individual to examine possible differences to the "IBS gut model".

One human clinical trial may be affected as follows:
A prospective double-blind, randomized, parallel group, pilot study in patients with D-IBS and/or C-IBS fixed dose study to evaluate the efficacy, safety and tolerability of a composition of the invention, e.g. comprising GOS in a range of about 5 to about 15 g per daily dose, e.g. about 10 g per daily dose, and Lactobacillus strain NCIMB 41114 in a concentration of about 10⁸ to about 10¹² cfu, e.g. about 10¹⁰ cfu per daily dose, e.g. corresponding to 2g of bacteria. The study consists of a 2-week baseline period, i.e. no composition or placebo is administered, a 12-week randomized double-blind treatment period with either placebo or the composition of the invention, followed by a 4-week withdrawal period. The efficacy, safety and tolerability of the composition of the invention is measured by an overall assessment of IBS symptoms after 12 weeks of treatment, determination of number of stool and consistency as described in the Bristol Stool Form Scale, assessment of abdominal discomfort or pain, intestinal bloating, feeling of urgency, straining or incomplete evacuation, nausea or flatulence. The microbial composition in the faeces of each patient is also assessed by selective plating, FISH, denaturing gradient gel electrophoresis (DGGE) and short chain fatty acid analysis during the pilot study.

The invention will now be further illustrated by the following Examples.

### Examples

### Example 1:

### Isolation of Lactobacillus strain NCIMB 41114

(i) Continuous culture system: Three chemostats are set up in a parallel; each is maintained under nitrogen gas, at 37°C, pH 6.5 and a dilution rate of 0.066h⁻¹. These conditions are set to resemble conditions typically found in the distal region of the human colon. The chemostats are fed with a control growth medium which comprised (gl⁻¹ in distilled water): yeast extract 2, peptone water 2, NaCl 0.1, K₂HPO₄ 0.04, KH₂PO₄ 0.04, MgSO₄.7H₂O 0.01, CaCl₂.H₂O 0.01, NaHCO₃ 2, Tween 80 2, hemin 0.05, Vitamin K1 0.01, cysteine.HCl 0.5, bile salts 0.5, glucose 0.4, starch 3, pectin 2 and arabinogalactan 1. The medium is autoclaved at 121°C for 30min and, whilst still hot, placed under nitrogen gas. After the medium has cooled, 1gl⁻¹ of filter sterilised tetracycline or 1gl⁻¹ of filter sterilised nystatin is aseptically added to the medium reservoir. Control chemostats with no antimicrobials added are also run.

(ii) Inoculation: Freshly voided fecal samples are obtained from healthy volunteers (n=6) and 10% (w/v) slurries anaerobically prepared using 0.1 mol I⁻¹ phosphate buffer pH 7 (26). None of the volunteers has any previous history of gastrointestinal disorder and has avoided antibiotics for at least 3 months prior to the study. The 300ml chemostat vessels are half filled with medium and 150ml of slurry added to each vessel. The system is then left for 24h to equilibrate before the medium pumps are started. The experiment is repeated with 6 different fecal donors in triplicate for each.

(iii) Microbial culture techniques: A sample is taken from each fecal slurry used as inocula and 1ml samples are also removed from each chemostat when the fermentation system has reached steady state (after 164h). These are then serially diluted (6-fold) with pre-reduced half strength peptone water. Each dilution is plated, in triplicate, onto pre-reduced (under an anaerobic, 10% CO₂, 10%H₂, 80%CO₂, atmosphere at 37°C) agars designed to select for predominant groups of gut bacteria: Wilkins Chalgren (Oxoid) for total anaerobes, Rogosa (Oxoid) for lactobacilli, Beerens for bifidobacteria, KVLB for bacteroides, Reinforced Clostridia agar (Oxoid) + novobocin 8mg/l and colostin 8mg/l for clostridia and Azide agar (Oxoid) for Gram positive cocci. Each dilution is then plated aerobically on to Nutrient Agar (Oxoid) to select for total aerobes, Sabouraud dextrose agar + chloramphenicol and cyclohexamide for yeast and MacConkey agar No.3 (Oxoid) for coliforms and incubated at 37°C. After 24-48h of incubation aerobic colonies are counted and after 48-72 h of incubation anaerobic plates are enumerated. Colonies with different morphotypes are also picked from the plates for gram stain, microscopic examination and phenotypic (biochemical) characterisation to confirm culture identities.

*Results:* Numbers of predominant gut microorganisms in the six inocula used are maintained after steady state conditions in the control chemostats. This confirms that the growth medium used is efficient at sustaining such populations in the continuous culture experiments. Hence, any differences in profiles resulting from antibiotic exposure are authentically due to these additions rather than any experimental variation. Yeasts are detected in 4 of the 6 volunteers faecal samples tested.

The effect of tetracycline is to markedly reduce populations of all bacteria tested, compared to controls, thereby confirming its broad spectrum of activity. One interesting observation is that the effect of tetracycline allows yeasts to increase in the fermentation systems in comparison to control chemostat levels. This has clinical implications for the use of tetracycline and associated risks with yeast overgrowth. In fact, in one of the inocula used here, yeasts are not initially detected, but are enriched for during the tetracycline chemostats.

As expected, yeasts are inhibited in the nystatin chemostats. However, certain bacterial genera are also affected. Principally, this involves a reduction in lactobacilli which are common probiotic microorganisms seen as important for gastrointestinal health. The clear indication is that nystatin usage is not conducive for the maintenance of indigenous probiotic levels.

During the tetracycline chemostat experiments, one of the runs produces an unexpected result. Yeasts are present at the start of the experiment but, contrary to the general trend observed, do not grow further. Instead a Gram positive rod predominated with no other cell types being detected. Genotypic work is therefore carried out to identify this microorganism.

### a) 16S rRNA sequencing

Universal 16S rRNA gene primer sets are used to amplify this gene from L. plantarum using PCR. This gene is specific for identifying bacteria and the variable region of this gene helps distinguish between bacterial species. 16S rRNA sequencing is carried out on the microorganism and the sequencing of a 1500 bp test yields the following sequence:

80 90 100 110 120 130
TTGAGTGAGTGGCGAACTGGTGAGTAACAC

140 150 160 170 180 190
GTGGGAAACCTGCCCAGAAGCGGGGATAACACCTGGAAACAGATGCTAATACCGCATAA

200 210 220 230 240 250
CAACTTGGACCGCATGGTCCGAGTTTGAAAGATGGCTTCGGCTATCACTTTTGGATGGTC

260 270 280 290 300 310
CCGCGGCGTATTAGCTAGATGGTGGGGTAACGGCTCACCATGGCAATGATACGTAGCCGA

320 330 340 350 360 370
CCTGAGAGGGTAATCGGCCACATTGGGACTGAGACACGGCCCAAACTCCTACGGGAGGCA

380 390 400 410 420 430
GCAGTAGGGAATCTTCCACAATGGACGAAAGTCTGATGGAGCAACGCCGCGTGAGTGAAG

440 450 460 470 480 490
AAGGGTTTCGGCTCGTAAAACTCTGTTGTTAAAGAAGAAC--ATATCTGAGAGTAACTGT

500 510 520 530 540 550
TCAGGTATTGACGGTATTTAACCAGAAAGCCACGGCTAACTACGTGCCAGCAGCCGCGGT

560 570 580 590 600 610
AATACGTAGGTGGCAAGCGTTGTCCGGATTTATTGGGCGTAAAGCGAGCGCAGGCGGTTT

620 630 640 650 660 670
TTTAAGTCTGATGTGAAAGCCTTCGGCTCAACCGAAGAAGTGCATCGGAAACTGGGAAAC

680 690 700 710 720 730
TTGAGTGCAGAAGAGGACAGTGGAACTCCATGTGTAGCGGTGAAATGCGTAGATATATGG

740 750 760 770 780 790
AAGAACACCAGTGGCGAAGGCGGCTGTCTGGTCTGTAACTGACGCTGAGGCTCGAAAGTA

800 810 820 830 840 850
TGGGTAGCAAACAGGATTAGATACCCTGGTAGTCCATACCGTAAACGATGAATGCTAAGT

860 870 880 890 900 910
GTTGGAGGGTTTCCGCCCTTCAGTGCTGCAGCTAACGCATTAAGCATTCCGCCTGGGGAG

920 930 940 950 960 970
TACGGCCGCAAGGCTGAAACTCAAAGGAATTGACGGGGGCCCGCACAAGCGGTGGAGCAT

980 990 1000 1010 1020 1030
GTGGTTTAATTCGAAGCTACGCGAAGAACCTTACCAGGTCTTGACATACTATGCAAATCT

1040 1050 1060 1070 1080 1090
AAGAGATTAGACGTTCCCTTCGGGGACATGGATACAGGTGGTGCATGGTTGTCGTCAGCT

1100 1110 1120 1130 1140 1150
CGTGTCGTGAGATGTTGGGTTAAGTCCCGCAACGAGCGCAACCCTTATTATCAGTTGCCA

1160 1170 1180 1190 1200 1210
GCATTAAGTTGGGCACTCTGGTGAGACTGCCGGTGACAAACCGGAGGAAGGTGGGGATGA

1220 1230 1240 1250 1260 1270
CGTCAAATCATCATGCCCCTTATGACCTGGGCTACACACGTGCTACAATGGATGGTACAA

1280 1290 1300 1310 1320 1330
CGAGTTGCGAACTCGCGAGAGTAAGCTAATCTCTTAAAGCCATTCTCAGTTCGGATTGTA

1340 1350 1360 1370
GGCTGCAACTCGCCTACATGAAGTCGGAATCGC

The microorganism is confirmed to be a *Lactobacillus* species closely related to Lactobacillus pentosus and Lactobacillus plantarum, which are phylogenetically similar. The Lactobacillus strain is subsequently deposited in accordance with the Budapest Treaty on 28 August 2001 at the NCIMB, Aberdeen, UK and receives accession number NCIMB 41114.

### b) Further identification

Further phenotypic characterization indicates that the organism is *Lactobacillus plantarum* through its ability to ferment D-xylose.
Protein profiling and rep-PCR independently confirm the identity as *Lactobacillus plantarum.* These methods allow taxonomic differentiation between *L. plantarum* and *L. pentosus.*

### c) Detection of L. plantarum using denaturing gradient gel electrophoresis (DGGE):

The technique of DGGE is used so that a unique marker for *L. plantarum* can be obtained when it is in a mixed community of bacteria i.e. when trying to identify it in a patient faecal sample. Specific Lactobacillus primers targeted a 340 bp region of the 16S rRNA gene with sufficient variation to distinguish all Lactobacillus groups. This amplified region (amplicon) is then run on a DGGE gel to elucidate a distinct pattern for the strain of interest. A ladder combining many *Lactobacillus* species may be used as a comparison in the analysis.

### Example 2: Production of Lactobacillus strain NCIMB 41114

For large-scale production of Lactobacillus strain NGIMB 41114 milk is used as a media. During spray drying pH is monitored and maintained above pH 5.5.

**Table 1 shows the growth of Lactobacillus strain NCIMB 41114 in milk.**

| Time (hours) | cfu/ml |
|---|---|
| 0 | 1.75E+08 |
| 2 | 2.00E+08 |
| 4 | 3.75E+08 |
| 6 | 4.125E+08 |
| 8 | 4.5E+08 |
| 10 | 3.75E+08 |

The cultures are assessed for viability after spray drying and are at the level of 2.8 x 10⁸ cfu/ml in 1g. After 1 month storage the viability is 2 x 10⁸ cfu/ml in 1g. After 2 months storage at room temperature the viability is 1 x 10⁸ cfu/ml in 1 g.

### Example 3: Characterization of oligosaccharide metabolism by Lactobacillus strain NCIMB 41114 : growth of Lactobacillus strain NCIMB 41114 in different prebiotics

### Method

The growth of Lactobacillus strain NCIMB 41114 in different prebiotics at a concentration of 1% (w/w) is monitored in order to see which of the oligosaccharides is better for its growth. The prebiotics that are used are the following: Fructooligosaccharide (FOS); Galactooligosaccharide (GOS); Xylooligosaccharide (XOS); Isomaltooligosaccharide (IMO); Soy-oligosaccharide (SOS). Glucose is used as a positive control, which is an efficient growth substrate but not selective or probiotics.

### Results

In order to justify the better substrate for the growth of Lactobacillus strain NCIMB 41114 the maximum growth rate is calculated from the growth curves obtained using the different prebiotics.

| Substrate | µ max |
|---|---|
| FOS | 0.09 |
| SOS | 0.15 |
| XOS | 0.06 |
| IMO | 0.10 |
| GOS | 0.12 |
| Glucose | 0.13 |

Soy-oligosaccharide (SOY or SOS), galactooligosaccharide (GOS) and isomaltooligosaccharide (IMO) show the best growth of Lactobacillus strain NCIMB 41114.

### Example 4: Batch culture investigations

### (a) Method

In order to monitor the growth of Lactobacillus strain NCIMB 41114 in mixed culture in the presence of different prebiotics batch culture fermentations are also performed. Such cultures are done in batch culture fermentaters. The fermenters are operated using physicochemical conditions that approximate those found in the proximal region of the colon, i.e. a slightly acidic pH, high substrate availability and volume. For this purpose the faecal samples of three different IBS volunteers are used and each experiment is done in duplicate. The prebiotics that are tested are the same as for the pure culture plus Benefiber® (BEN) and mixture of Benefiber® (BEN) with GOS (1:2). All the prebiotics are added in a concentration of 1%. In the batch culture fermenters the pH and temperature are kept constant at 6.8 and 37°C respectively.
Lactobacillus strain NCIMB 41114 is added at a concentration of 1% of an overnight culture (10⁷ cells/ml) in each fermenter. Sample are taken at 0, 5, 10 and 24 hours of fermentation for plating out, i.e. for monitoring the growth of the probiotic, for F.I.S.H., i.e. enumeration of different bacterial groups, and short chain fatty acids analysis.

### (b) Results

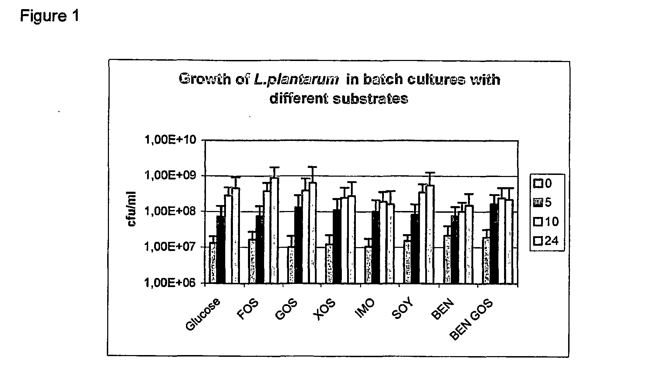

Figure 1 shows the growth of the probiotic during the fermentation

A potentially higher increase of Lactobacillus strain NCIMB 41114 after 24 hours of fermentation is observed with the use of FOS, GOS and SOS (SOY), respectively.

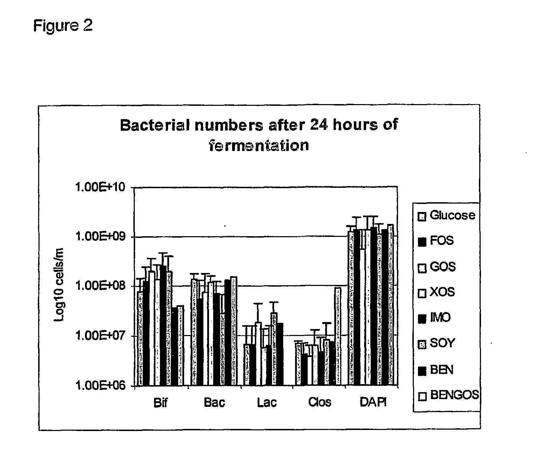

Figure 2 shows the bacterial numbers (FISH counts) achieved after 24 hours of fermentation in the batch cultures.

The results suggest that IMO, SOS (SOY) and GOS show best growth of bifidobacteria whereas SOS (SOY), BEN and GOS show best growth of Lactobacilli. GOS shows the lowest growth of Clostridia.

### Example 5:

Package comprising:
- 1 sachet comprising 2 g lyophilized Lactobacillus strain NCIMB 41114 (10¹⁰ cfu)
- 1 sachet comprising:
   GOS 10 g
   Lactose 3.6 g

Both sachets are to be dissolved in approximately 200 ml of cold orange juice, just before consumption. 1 serving per day.

### Example 6:

Compositions are prepared by intimately admixing the prebiotics with 2g of the spray dried Lactobacillus strain NCIMB 41114 (NCIMB 41114) to obtain a powder. The powder is dissolved in 250 ml of water to obtain a ready-to-drink dietary supplement.

| Example | 6A | 6B | 6C | 6D | 6E | 6F |
|---|---|---|---|---|---|---|
| Prebiotic (g) | | | | | | |
| GOS | 9 | 10 | 5 | 4.3 | 11.25 | 10 |
| FOS | | | 5 | 4.3 | | |

| Benefiber®⁽¹⁾ | 3 | 1 | 1 | 1.4 | 3.75 | |
|---|---|---|---|---|---|---|
| Probiotic | | | | | | |
| (cfu/g) | 10¹⁰ | 10¹⁰ | 10¹⁰ | 10¹⁰ | 10¹⁰ | 10¹⁰ |
| NCIMB 41114 | | | | | | |

| | | | | | | |
|---|---|---|---|---|---|---|
| ⁽¹⁾hydrolyzed guar gum | | | | | | |

Other examples may be made by replacing GOS and/or FOS by SOS, IMO or any of the prebiotics specified hereinabove.

### Example 7:

Ready-to-drink nutritional composition made by dissolving the content of sachets 1 and 2 in 250 ml of water:

| Sachet 1: | |
|---|---|
| FOS | 4.4g |
| GOS | 4.4g |
| Glutamine | 3.4g |
| EPA | 1g |
| DHA | 0.5g |

| Sachet 2: | |
|---|---|
| Probiotic (1) (10¹⁰ cfu) | 2g |
| Carbohydrates | 12.9g |

| | |
|---|---|
| ⁽¹⁾ spray dried Lactobacillus strain NCIMB 41114. | |

The examples illustrate compositions useful f or example a s alternatives or adjuncts to conventional therapy for any of the disease described hereinabove in that they stimulate a beneficial gut flora composition on administration of e.g. one dose per day, e.g. split in two servings.

## Claims

1. A composition comprising Lactobacillus strain NCIMB 41114 and at least one non-digestible oligosaccharide.

2. A composition according to claim 1 wherein said oligosaccharide is chosen from galacto-oligosaccharide (GOS), fructo-oligosaccharide (FOS), xylo-oligosaccharide (XOS), soybean-oligosaccharide (SOS), isomalto-oligosaccharide (IMO), gentio-oligosaccharide, fructan, inulin, lactulose, gluco-oligosaccharide and lactosucrose.

3. A composition according to claim 1 or claim 2 which in a daily dosage provides from 10⁶ to 10¹² cfu Lactobacillus strain NCIMB 41114.

4. A composition according to any one of claims 1 to 3 wherein said composition comprises FOS and/or GOS.

5. A composition according to any one of claims 1 to 4 wherein said composition further comprises a soluble fiber selected from the group consisting of guar gum and partially hydrolyzed guar gum.

6. A composition according to any one of claims 1 to 5 wherein said composition further comprises at least one polyunsaturated fatty acid chosen from at least one of alpha-linolenic acid, eicosapentaenoic acid, docosapentaenoic acid or docosahexaenoic acid.

7. A composition according to claim 6 wherein said polyunsaturated fatty acid comprises eicosapentaenoic acid and/or docosahexaenoic acid.

8. A composition according to any preceding claim for use as a medicament.

9. Use of a composition according to any one of claims 1 to 8 in the manufacture of a medicament or nutritional composition for any of maintaining and/or promoting a healthy gut microflora, reducing the toxic effects of the digestive process, stimulating the digestive system and improving bowel control.

10. Use of a composition according to any one of claims 1 to 8 in the manufacture of a medicament or nutritional formulation for the prevention or treatment of any form of Functional Bowel Disorder, and in particular functional constipation, functional diarrhea and Irritable Bowel Syndrome (IBS), such as Constipation predominant IBS, Alternating IBS and Diarrhea predominant IBS.

11. Use of a composition according to any one of claims 1 to 8 in the manufacture of a medicament or nutritional formulation for stimulating the immune system and promoting resistance to bacterial or yeast infections, in particular for the prevention or treatment of Candidlasis or diseases induced by sulfate-reducing bacteria.

12. Use of a composition according to any one of claims 1 to 8 in the manufacture of a medicament or nutritional composition for the prevention or treatment of Inflammatory Bowel Disease, in particular Ulcerative Colltis, Crohn's disease, and/or to prevent colon cancer.

13. A commercial package comprising as active agents lactobacillus strain NCIMB 41114 and at least one non-digestible oligosaccharide together with instructions for simultaneous, separate or sequential use thereof in maintaining and/or restoring a beneficial gut microflora, for the prevention or treatment of any form of Functional Bowel Disorder, and In particular Irritable Bowel Syndrome (IBS), for eliminating sulfate reducing bacteria, for the treatment or prevention of Inflammatory Bowel Disease (IBD), in particular Ulcerative Colitis, Crohn's disease, and/or colon cancer, and/or for repressing or prolonging the remission periods on Ulcerative patients.

14. A package according to claim 13 wherein said oligosaccharide is chosen from galacto-oligosaccharide (GOS), fructo-oligosaccharlde (FOS), xylo-oligosaccharide (XOS), soybean-oligosaccharide (SOS), isomalto-oligosaccharide (IMO), gentio-oligosaocharide, fructan, inulin, lactulose, gluco-oligosaccharide and lactosucrose.

## Patentansprüche

1. Zusammensetzung, die den Lactobacillus-Stamm NCIMB 41114 und mindestens ein nicht-verdaubares Oligosaccharid enthält.

2. Zusammensetzung nach Anspruch 1, worin das Oligosaccharid ausgewählt ist unter Galacto-Oligosaccharid (GOS), Fructo-Oligosaccharid (FOS), Xylo-Oligosaccharid (XOS), Sojabohnen-Oligosaccharid (SOS), Isomalto-Oligosaccharid (IMO), Gentio-Oligosaccharid, Fructan, Inulin, Lactulose, Gluco-Oligosaccharid und Lactosaccharose.

3. Zusammensetzung nach Anspruch 1 oder Anspruch 2, welche in einer täglichen Dosis von 10⁶ bis 10¹² cfu des Lactobacillen-Stamms NCIMB 41114 liefert.

4. Zusammensetzung nach einem der Ansprüche 1 bis 3, worin die Zusammensetzung FOS und/oder GOS umfasst.

5. Zusammensetzung nach einem der Ansprüche 1 bis 4, worin die Zusammensetzung weiter eine lösliche Faser umfasst, ausgewählt aus der Gruppe bestehend aus Guar-Gum und teilweise hydrolisiertem Guar-Gum.

6. Zusammensetzung nach einem der Ansprüche 1 bis 5, worin die Zusammensetzung weiter mindestens eine mehrfach ungesättigte Fettsäure umfasst, ausgewählt unter mindestens einer von alpha-Linolensäure, Eicosapentaensäure, Docosapentaensäure oder Docosahexaensäure.

7. Zusammensetzung nach Anspruch 6, worin die mehrfach ungesättigte Fettsäure Eicosapentaensäure und/oder Docosahexaensäure umfasst.

8. Zusammensetzung nach einem der vorstehenden Ansprüche zur Verwendung als Medikament.

9. Verwendung einer Zusammensetzung nach einem der Ansprüche 1 bis 8 zur Herstellung eines Medikaments oder einer Nahrungsmittelzusammensetzung zu jeglichem aus der Aufrechterhaltung und/oder Förderung einer gesunden Darmmikroflora, der Reduzierung der toxischen Auswirkungen von Verdauungsprozessen, der Stimulierung des Verdauungssystems und der Förderung der Darmsteuerung.

10. Verwendung einer Zusammensetzung nach einem der Ansprüche 1 bis 8 zur Herstellung eines Medikaments oder einer Nahrungsformulierung zur Verhinderung oder Behandlung jeder Form von funktioneller Darmstörung und insbesondere funktioneller Darmträgheit, funktioneller Diarrhöe und Reizdarmsyndrom (IBS), wie IBS mit prädominanter Darmträgheit, alternierendes IBS und IBS mit prädominanter Diarrhöe.

11. Verwendung einer Zusammensetzung nach einem der Ansprüche 1 bis 8 zur Herstellung eines Medikaments oder einer Nahrungsmittelzusammensetzung zur Stimulierung des Immunsystems und zur Förderung der Widerstandsfähigkeit gegen bakterielle oder Hefe-Infektionen, insbesondere zur Verhinderung oder Behandlung von Candidiasis oder Erkrankungen, die durch Sulfat-reduzierende Bakterien induziert sind.

12. Verwendung einer Zusammensetzung nach einem der Ansprüche 1 bis 8 zur Herstellung eines Medikaments oder einer Nahrungsmittelzusammensetzung zur Verhinderung oder Behandlung von entzündlicher Darmerkrankung, insbesondere eiternde Kolitis (Colitis ulcerosa), Crohn's Erkrankung, und/oder zur Verhinderung von Darmkrebs.

13. Handelsverpackung, die als aktive Inhaltsstoffe den Lactobacillus Stamm NCIMB 41114 und mindestens ein nicht-verdaubares Oligosaccharid zusammen mit Instruktionen zur gleichzeitigen, separaten oder sequenziellen Verwendung davon umfasst, zur Aufrechterhaltung und/oder Wiederherstellung einer günstigen Darmmikroflora, zur Verhinderung oder Behandlung jeder Form funktioneller Darmstörung, und insbesondere Reizdarmsyndrom (IBS), zur Eliminierung von Sulfat-reduzierenden Bakterien, zur Behandlung oder Verhinderung von entzündlicher Darm-Erkrankung (IBD), insbesondere eiternder Kolitis (Colitis ulcerosa), Crohn's Erkrankung und/oder Darm-Krebs, und/oder zur Unterdrückung oder Verlängerung der Zeitspannen des Wiederauftretens bei Ulcer-Patienten.

14. Handelsverpackung nach Anspruch 13, worin das Oligosaccharid ausgewählt ist unter Galacto-Oligosaccharid (GOS), Fructo-Oligosaccharid (FOS), Xylo-Oligosaccharid (XOS), Sojabohnen-Oligosaccharid (SOS), Isomalto-Oligosaccharid (IMO), Gentio-Oligosaccharid, Fructan, Inulin, Lactulose, Gluco-Oligosaccharid und Lactosaccharose.

## Revendications

1. Composition comprenant la souche de Lactobacillus NCIMB 41114 et au moins un oligosaccharide non digestible.

2. Composition selon la revendication 1, dans laquelle ledit oligosaccharide est choisi parmi un galacto-oligosaccharide (GOS), un fructo-oligosaccharide (FOS), un xylo-oligosaccharide (XOS), un oligosaccharide du soja (SOS), un isomalto-oligosaccharide (IMO), un gentio-oligosaccharide, le fructane, l'inuline, le lactulose, un gluco-oligosaccharide et le lactosucrose.

3. Composition selon la revendication 1 ou la revendication 2 qui, dans une posologie quotidienne, procure entre 10⁶ et 10¹² cfu de la souche de Lactobacillus NCIMB 41114.

4. Composition selon l'une quelconque des revendications 1 à 3, ladite composition comprenant un FOS et/ou un GOS.

5. Composition selon l'une quelconque des revendications 1 à 4, ladite composition comprenant en outre une fibre soluble choisie dans le groupe constitué par la gomme guar et la gomme guar partiellement hydrolysée.

6. Composition selon l'une quelconque des revendications 1 à 5, ladite composition comprenant en outre au moins un acide gras polyinsaturé choisi parmi au moins l'un de l'acide alpha-linolénique, l'acide eicosapentaénoïque, l'acide docosapentaénoïque et l'acide docosahexaénoïque.

7. Composition selon la revendication 6, dans laquelle ledit acide gras polyinsaturé comprend l'acide eicosapentaénoïque et/ou l'acide docosahexaénoïque.

8. Composition selon l'une quelconque des revendications précédentes, destinée à être utilisée en tant que médicament.

9. Utilisation d'une composition selon l'une quelconque des revendications 1 à 8, dans la fabrication d'un médicament ou d'une composition nutritionnelle destiné(e) à l'un quelconque parmi maintenir et/ou favoriser une microflore intestinale bénéfique, réduire les effets toxiques du processus digestif, stimuler le système digestif et améliorer le contrôle de l'intestin.

10. Utilisation d'une composition selon l'une quelconque des revendications 1 à 8, dans la fabrication d'un médicament ou d'une formulation nutritionnelle pour la prévention ou le traitement d'une quelconque forme de trouble fonctionnel de l'intestin, et en particulier une constipation fonctionnelle, une diarrhée fonctionnelle et un syndrome du côlon irritable (IBS), tel qu'un IBS avec constipation prédominante, un IBS d'alternance et un IBS avec diarrhée prédominante.

11. Utilisation d'une composition selon l'une quelconque des revendications 1 à 8, dans la fabrication d'un médicament ou d'une formulation nutritionnelle pour stimuler le système immunitaire et promouvoir une résistance contre des infections bactériennes ou dues à des levures, en particulier pour la prévention ou le traitement d'une candidose ou de maladies induites par des bactéries réduisant les sulfates.

12. Utilisation d'une composition selon l'une quelconque des revendications 1 à 8, dans la fabrication d'un médicament ou d'une composition nutritionnelle pour la prévention ou le traitement de maladies inflammatoires de l'intestin, en particulier une rectocolite ulcéro-hémorragique, la maladie de Crohn, et/ou pour prévenir un cancer du côlon.

13. Conditionnement commercial comprenant en tant qu'agents actifs la souche de Lactobacillus NCIMB 41114 et au moins un oligosaccharide non digestible en même temps que des instructions pour leur utilisation simultanée, séparée ou successive dans le maintien et/ou la restauration d'une microflore intestinale bénéfique, pour la prévention ou le traitement d'une quelconque forme de trouble fonctionnel de l'intestin, et en particulier d'un syndrome du côlon irritable (IBS), pour l'élimination de bactéries réduisant les sulfates, pour le traitement ou la prévention d'une maladie inflammatoire de l'intestin (IBD), en particulier une rectocolite ulcéro-hémorragique, la maladie de Crohn, et/ou un cancer du côlon, et/ou pour ralentir ou prolonger les périodes de rémission chez des patients ayant un ulcère.

14. Conditionnement selon la revendication 13, dans lequel ledit oligosaccharide est choisi parmi un galacto-oligosaccharide (GOS), un fructo-oligosaccharide (FOS), un xylo-oligosaccharide (XOS), un oligosaccharide du soja (SOS), un isomalto-oligosaccharide (IMO), un gentio-oligosaccharide, le fructane, l'inuline, le lactulose, un gluco-oligosaccharide et le lactosucrose.
